(19) Europäisches Patentamt
European Patent Office
Office européen des brevets

(11) EP 1 233 763 B1

(12) EUROPÄISCHE PATENTSCHRIFT

(45) Veröffentlichungstag und Bekanntmachung des Hinweises auf die Patenterteilung:
**30.07.2003 Patentblatt 2003/31**

(21) Anmeldenummer: **00985090.0**

(22) Anmeldetag: **24.11.2000**

(51) Int Cl.$^7$: **A61K 9/70**

(86) Internationale Anmeldenummer:
**PCT/EP00/11692**

(87) Internationale Veröffentlichungsnummer:
**WO 01/039753 (07.06.2001 Gazette 2001/23)**

(54) **TRANSDERMALE THERAPEUTISCHE SYSTEME MIT VERBESSERTER STABILITÄT UND EIN VERFAHREN ZU IHRER HERSTELLUNG**

TRANSDERMAL THERAPEUTIC SYSTEMS WITH IMPROVED STABILITY AND A METHOD FOR THE PRODUCTION THEREOF

SYSTEMES THERAPEUTIQUES TRANSDERMIQUES AYANT UNE STABILITE AMELIOREE ET PROCEDE DE FABRICATION

(84) Benannte Vertragsstaaten:
**AT BE CH CY DE DK ES FI FR GB GR IE IT LI LU MC NL PT SE TR**

(30) Priorität: **29.11.1999 DE 19957401**
**04.11.2000 DE 10054713**

(43) Veröffentlichungstag der Anmeldung:
**28.08.2002 Patentblatt 2002/35**

(73) Patentinhaber: **LTS Lohmann Therapie-Systeme AG**
**56626 Andernach (DE)**

(72) Erfinder: **MÜLLER, Walter**
**56564 Neuwied (DE)**

(74) Vertreter: **Schmidt, Werner, Dr. et al**
**LTS LOHMANN Therapie-Systeme AG,**
**Lohmannstrasse 2**
**56626 Andernach (DE)**

(56) Entgegenhaltungen:
**EP-A- 0 304 381**
**EP-A- 0 441 333**
**EP-A- 0 965 626**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 233 763 B1

## Beschreibung

Transdermale Therapeutische Systeme (TTS) können unter Vernachlässigung von wenig gebräuchlichen Sonderformen in zwei Grundgruppen unterschieden werden, die sogenannten Matrixsysteme und die sogenannten Reservoirsysteme. Bei den sogenannten Matrixsystemen ist im einfachsten Fall der Wirkstoff in einer selbstklebenden Schicht gelöst bzw. zum Teil auch nur in Form von Kristallen suspendiert. Reservoirsysteme stellen eine Art Beutel aus einer inerten Rückschicht und einer wirkstoffdurchlässigen Membran dar, wobei sich der Wirkstoff in einer flüssigen Zubereitung in diesem Beutel befindet. Meistens ist die Membran mit einer Kleberschicht versehen, die der Verankerung des Systems auf der Haut dient.

Unabhängig von speziellen Ausführungsformen des transdermalen Systems wird der Wirkstoff durch Diffusion bei Gebrauch an die Haut abgegeben und muß deshalb zumindest zum Teil in gelöster Form vorliegen.

In dieser Form ist der Wirkstoff besonders empfindlich für Reaktionen mit Bestandteilen der Formulierung, die zur Beeinträchtigung der Stabilität führen können. Solche Reaktionen sind z. B.:

a) die Bindung des Wirkstoffs über eine Amid- oder Esterbindung an Carboxyl- oder Estergruppen der verwendeten Polymere oder Permeationsenhancern;
b) die Reaktion einer Carboxyl- oder Estergruppe des Wirkstoffs mit alkoholischen Gruppen von klebrigmachenden Harzen oder Permeationsenhancern;
c) die Hydrolyse bzw. Alkoholyse von Estergruppen durch Wasser bzw. Alkohole.

Solche Reaktionen, die sich unmittelbar ableiten lassen aus den funktionellen Gruppen des Wirkstoffs und der Hilfsstoffe sind für den Fachmann keine Überraschung. Entsprechende Stabilitätsrisiken werden deshalb gewöhnlich sehr schnell durch entsprechende Kompatibilitätsuntersuchungen bei erhöhter Temperatur aufgedeckt und können dann durch entsprechende Umformulierungen ungünstiger Wirkstoff-Hilfsstoffkombinationen vermieden werden.

Desweiteren kann die Stabilität des Wirkstoffs und der Hilfsstoffe durch die Reaktion mit aktivem Sauerstoff gefährdet sein.
Solch aktiver Sauerstoff ist natürlich der Sauerstoff der Luft. Ein wirksames Mittel, den im TTS enthaltenen Wirkstoff gegen diesen Sauerstoff zu schützen ist es, das TTS unter einer Stickstoffatmosphäre zu verpacken und/oder Antioxidantien zusätzlich in die Packung hineinzugeben.

Trotz dieser Vorsichtsmaßnahmen mußte man bisher jedoch bei längerer Lagerung eines TTS mit oxidationsempfindlichen Wirkstoffen oftmals eine mehr oder weniger starke Abnahme des Wirkstoffgehalts in Kauf nehmen. Die Ursachen dafür waren bisher nicht bekannt.

Es wurde nun überraschenderweise gefunden, daß Rohstoffe, die für die Herstellung von TTS benutzt werden, in erheblichem Maße aktiven Sauerstoff in einer anderen Form, nämlich in Form von Hydroperoxiden, enthalten können.

Diese Hydroperoxide können sich gemäß der in der Literatur beschriebenen Autoxidationsreaktionen nach folgenden Mechanismus bilden:

$$R{-}H \rightarrow R^{\cdot} + H^{\cdot} \qquad \text{Gl. 1a}$$

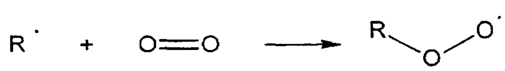
Gl. 1b

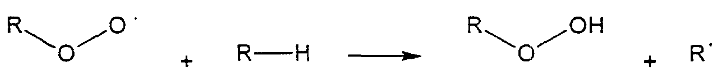
Gl. 1c

Im ersten Schritt, der sogenannten Induktionsphase, werden durch Einwirkung von Wärme, Licht unter Begünstigung von Schwermetallspuren unter Verlust eines Wasserstoffatoms freie Radikale gebildet.
Im zweiten Schritt, der sogenannten Propagationsphase, reagieren diese Radikale mit Sauerstoff unter Bildung von Peroxyradikalen.
Diese Peroxyradikale greifen nun weitere Moleküle an unter Bildung von Hydroperoxiden und einem neuen freien

Radikal. Damit hat eine Kettenreaktion eingesetzt, die solange weiterläuft bis diese Kette durch die Reaktion von zwei Radikalen miteinander, wie z.B. in untenstehender Gleichung dargestellt, abbricht.

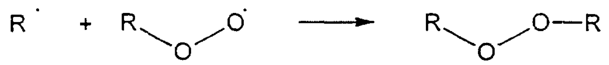

Gl. 1d

Das als Kettenüberträger fungierende Peroxidradikal greift aufgrund seiner relativ geringen Reaktivität besonders an den Stellen an, die zu einem energiearmen Radikal am Substrat führen. Solche bevorzugten Stellen sind C-H-Bindungen in Benzyl- oder Allylstellung, tertiäre C-H-Bindungen und C-H-Bindungen in Nachbarschaft von Ethersauerstoffen. Damit sind speziell solche Rohmaterialien für die Bildung von Hydroperoxiden empfänglich, die über solche Gruppen verfügen.

Die zum Schutz oxidationsempfindlicher Wirkstoffe eingesetzten Antioxidantien bzw. Stabilisatoren können in diese Reaktionskette eingreifen. Antioxidantien können unterschieden werden in sogenannte Radikal- und Sauerstofffänger. Radikalfänger wie z.B. Tocopherol und seine Derivate entfernen bzw. inaktivieren Radikale und unterbrechen dadurch den Kettenmechanismus der Autoxidation. Sauerstofffänger, wie z.B. Ascorbylpalmitat, reagieren direkt mit dem oxidativen Agens und verhindern so den Start der Kettenreaktion.

Der Zusatz von Antioxidantien/Stabilisatoren ist jedoch nur dann sinnvoll, wenn nicht schon die Ausgangsmaterialien oxidativ wirkenden Hydroperoxide enthalten und die Arzneiform durch die Verpackung vor dem Zutritt von Sauerstoff geschützt ist.

Überraschenderweise konnte festgestellt werden, daß es in allen für die Herstellung von transdermalen therapeutischen Systemen verwendeten Rohstoffklassen mit Ausnahme der folienförmigen Materialien Vertreter gibt, die schon bei Anlieferung bzw. kurzzeitiger Lagerung mit erheblichen Mengen an Hydroperoxiden belastet sind. Konkret heißt das, daß Polymere, klebrigmachende Harze, Permeationsenhancer und Lösemittel bzw. Löslichkeitsvermittler einen Hydroperoxidgehalt haben können, der in erheblichem Maße die Stabilität eines oxidationsempfindlichen Wirkstoffs beeinträchtigen kann.

Gewöhnlich wird der Gehalt an Peroxiden durch die sogenannte Peroxidzahl POZ ausgedrückt, die die Menge an Milliequivalenten aktiven Sauerstoffs pro kg Substanz angibt. Für die Bestimmung der Peroxidzahl gibt es verschiedene Methoden. Am gebräuchlichsten ist die Umsetzung einer definierten Menge Substanz in einer Chloroform/ Eisessig-Lösung mit einem Überschuß an Jodidionen und anschließender Rücktitration des gebildeten Jods mit Natriumthiosulfat. Weniger gebräuchlich und auf wässrige Lösungen beschränkt ist die Umsetzung der Substanz mit Titan (IV)-ionen und die photometrische Vermessung des sich bildenden Peroxokomplexes.

Besonders leicht durchzuführen ist ein semiquantitativer Test auf Peroxide mit käuflichen Teststäbchen.

In der untenstehender Tabelle sind die gemessene Peroxidzahlen von einigen exemplarischen, für die Herstellung von Reservoir- und Matrixsystemen genutzten Substanzen nach ca. 6 Monaten Lagerung bei Raumtemperatur aufgelistet. Die Peroxidzahlen wurden gemäß den beiden zuerst erwähnten Methoden gemessen.

| Rohstoff | Funktion | POZ |
|---|---|---|
| Kohlenwasserstoffharz | Matrixbestandteil | 180 |
| Kollidon | Matrixbestandteil | 110 |
| partiell hydrierter Glycerolester von Kolophonium | Klebrigmacher | 190 |
| hydrierter Glycerolester von Kolophonium | Klebrigmacher | 80 |
| Poly-β-pinen | Klebrigmacher | 150 |
| Dieethylenglykolmonoethylether | Lösemittel/ Permeationsenhancer | 120 |
| Oleylalkohol | Lösemittel/ Permeationsenhancer | 50 |
| Limonen | Permeationsenhancer | 15 |

Die Peroxidzahl der fertigen Pflaster kann nach der gleichen Methode bestimmt werden. Allerdings ist es etwas schwierig, genügend Pflaster in einer nicht zu großen Menge Chloroform zu lösen.

Einfacher ist es, die Peroxidbelastung der Einzelstoffe zu messen und die Peroxidzahl des wirkstoffhaltigen Teils der Pflaster nach folgender Formel zu berechnen:

$$\sum_{i=1}^{n} ( N_i \bullet POZ/100)$$

n : Anzahl der Formulierungsbestandteile des wirkstoffhaltigen Teils des Systems

N : prozentualer Gehalt der Formulierungsbestandteile in den wirkstoffhaltigen Bestandteilen des Systems (numerischer Wert)

POZ : Peroxidzahl der Einzelbestandteile des wirkstoffhaltigen Teils des Systems Experimentell wurde gefunden, daß die in den Rohstoffen enthaltenen Hydroperoxide auf vielfältige Art und Weise mit dem mit ihnen in Kontakt kommenden Wirkstoff reagieren können. Als besonders empfindlich haben sich dabei Wirkstoffe gezeigt, die über eine der folgenden Teilstrukturen verfügen:

- •sekundäre oder tertiäre Aminogruppen
- •C-C- Doppelbindungen
- •C-H-Gruppen in Allylstellung
- •benzylische C-H-Gruppen
- •tertiäre C-H-Gruppen
- •Sulfid oder Sulfoxidgruppen

Die entsprechenden Reaktionsprodukte sehen dabei wie folgt aus:

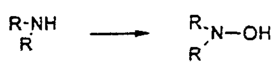

Gl. 2a

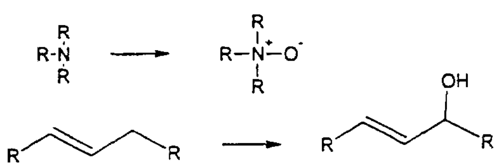

Gl. 2b

Gl. 2c

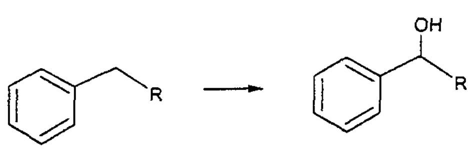

Gl. 2d

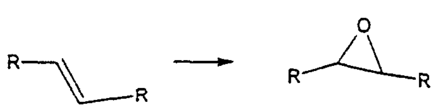

Gl. 2e

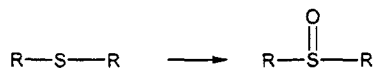

Gl. 2f

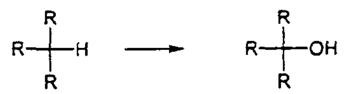

Gl. 2g

R = organischer Rest

In vielen Fällen sind diese Reaktionen an den entsprechenden funktionellen Gruppen der Wirkstoffe von Folgereaktionen begleitet.

So wurde z.B. gefunden, daß im Falle des 17-β-Estradiols zuerst eine Hydroxylierung in der Benzylposition ( C 9) erfolgt, wobei die Hydroxylgruppe dann als Wasser unter Bildung von Δ 9(11) 17-β-Estradiol eliminiert wird. Diese Reaktion ist begünstigt, da dadurch eine konjugierte Doppelbindung entsteht.

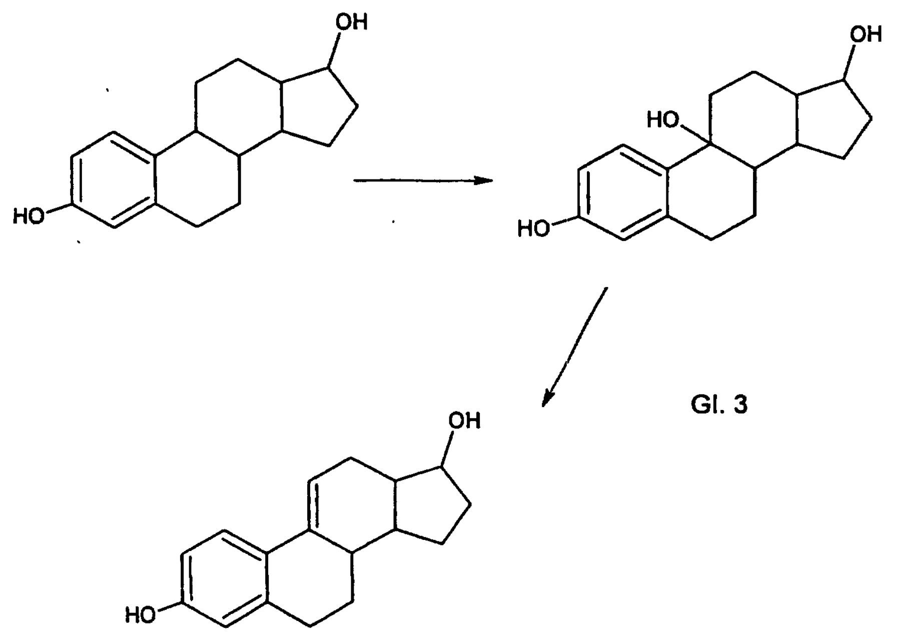

Gl. 3

Im Falle eines Wirkstoffs mit einem aminsubstituierten Tetrahydronaphtolfragment ( N- 0923 ) wurde gefunden, daß sich zuerst ein N-Oxid bildet, das dann in einer Eliminierungsreaktion ( Cope-Eliminierung ) gemäß untenstehendem Reaktionsschema weiterreagiert zu einem Dihydronaphtol und einem Hydroxylamin.

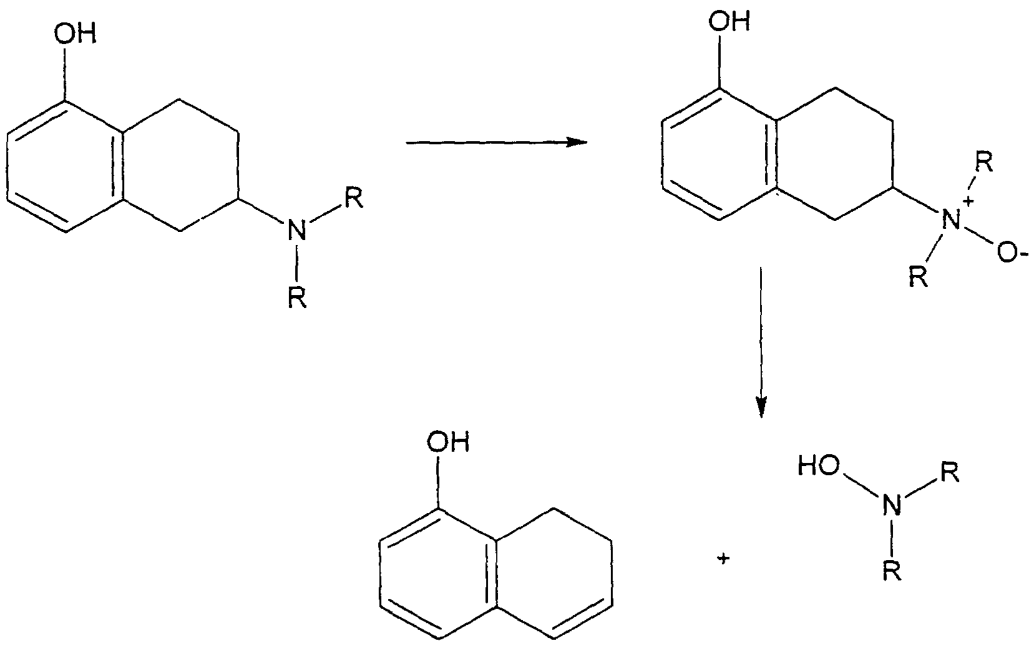

Gl. 4

Bei Calciumantagonisten vom Typ der Dihydropyridine wurde folgender Abbaumechanismus gefunden.

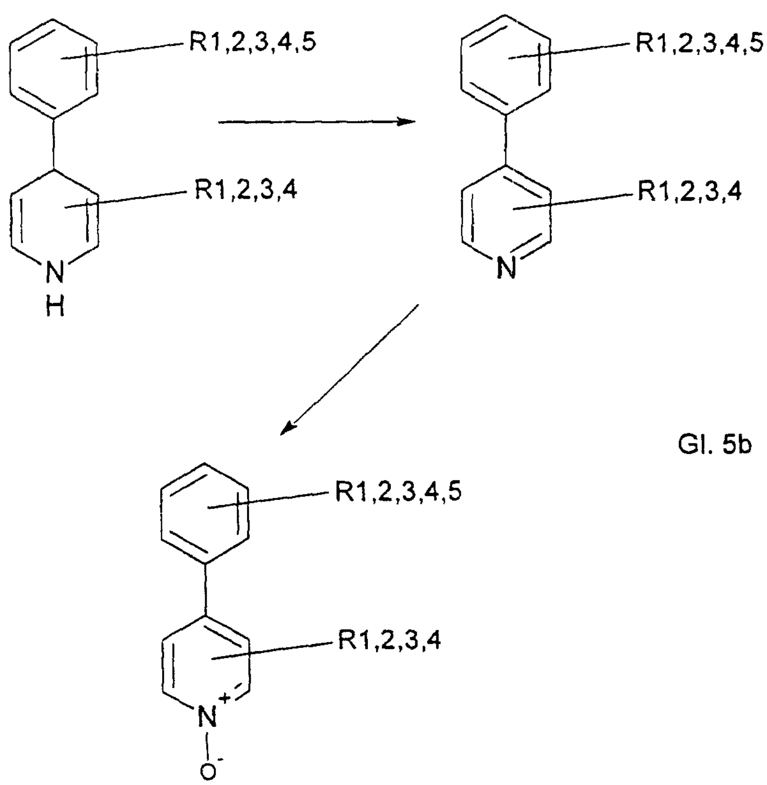

Gl. 5a

Gl. 5b

Es ist unklar ob der erste Angriff am Stickstoff oder der tertiären C-H-Bindung des Dihydropyridinrings erfolgt. Auf jeden Fall folgt auch hier eine Wasserabspaltung, die wegen der Ausbildung eines aromatischen Zustands energetisch begünstigt ist.

Die Weiterreaktion zum N-Oxid nach erfolgter Oxidation des Dihydropyridinrings wird nur in der Reaktion mit t-Butylhydroperoxid gemäß Gleichung 5b beobachtet. In Pflastersystemen ist die gebildete Menge zu klein, um bei geringen Umsätzen beobachtet zu werden.

Die oben geschilderten Beispiele zeigen, daß an den Reaktionsprodukten oft nicht erkennbar ist, daß an der Abbaureaktion unmittelbar Hydroperoxide beteiligt sind. Für die Ermittlung der Empfindlichkeit des Wirkstoffs für Oxidationsreaktionen mit Hydroperoxiden wurde eine einfach durchzuführende Testreaktion entwickelt. Dazu wird der Wirkstoff in Chloroform oder einem anderen geeigneten Lösemittel mit t-Butylhydroperoxid unter Rückfluß umgesetzt. Werden in dieser Reaktionsmischung oxidative Abbauprodukte des Wirkstoffs gefunden, sind sie auf die Reaktion mit dem Hydroperoxid zurückzuführen. Oftmals erkennt man einen Abbau des Wirkstoffs auch sehr einfach an einer Verfärbung der Testlösung. Aus einem positiven Ausgang der Testreaktion ist die praktische Schlußfolgerung zu ziehen, daß bei der Formulierung eines transdermalen Systems mit diesem Wirkstoff nur solche Hilfsstoffe zu verwenden sind, die weitgehend frei von Hydroperoxiden sind.

Die der vorliegenden Erfindung zugrundeliegende Aufgabe besteht darin, ein transdermales therapeutisches System (TTS) bereitzustellen, in dem die Bildung von oxidativen Abbauprodukten der in diesem TTS enthaltenden oxidationsempfindlichen Wirkstoffen während der Lagerung des TTS verringert ist. Die Lösung dieser Aufgabe liegt, wie bereits vorstehend ausgeführt, darin, bei der Herstellung eines TTS, welches einen oder mehrere oxidationsempfindliche Wirkstoffe enthält, nur solche Formulierungsbestandteile zu verwenden, die weitgehend frei von Hydroperoxiden sind. Erfindungsgemäß sind das solche Formulierungsbestandteile, die zusammen in den durch die Rezeptur für das TTS gegebenen Anteilen eine Peroxidzahl (POZ) von maximal 20, bevorzugt von maximal 10, besonders bevorzugt von maximal 5 aufweisen.

Unter dem Begriff Formulierungsbestandteile sind alle Stoffe des TTS- außer dem oder den pharmazeutischen Wirkstoffen - zu verstehen, in denen der oder die Wirkstoff(e) enthalten sind.

Dazu gehören: Als Bausteine der ein- oder mehrschichtigen Matrix bzw. des Reservoirsystems: z. B. Polymere von Kohlenwasserstoffen wie Polyethylene, Polypropylene, Polyacrylate, Polymethacrylate, Polyurethane, Polyisobutylene, Polyvinylpyrolidon; Kohlenwasserstoffharze; Silicone; Kautschuk; Copolymere des Vinylpyrrolidons mit Acrylsäuren, Acrylsäurederivaten, Ethylen und/oder Vinylacetat; Harze auf der Basis von Kolophoniumderivaten und/oder Polyterpenen.

Als funktionale Zusatzstoffe oder Hilfsstoffe: z. B. Weich- bzw. Klebrigmacher wie Kolophoniumester, z. B. hydrierte oder partiell hydrierte Glyzerolester von Kolophonium, Polyterpene; Permeationsenhancer wie z. B. Terpene oder Terpenderivate, ungesättigte Fettsäuren oder deren Derivate, Ester von langkettigen Fettsäuren, Diethylenglykol oder dessen Derivate; Alkylmethylsulfoxide, Azone und Limonene; Kristallisationsinhibitoren wie z. B. Polyvinylpyrrolidon; Polyacrylsäure oder Cellulosederivate; Lösungsmittel wie z. B. Polyethylenglykol, Diethylenglykol und/oder dessen Derivate, Propandiol oder Oleylalkohol.

Falls Formulierungsbestandteile, welche für die Herstellung von transdermalen therapeutischen Systemen mit oxidationsempfindlichen Wirkstoffen bestimmt sind, schon bei Anlieferung mit erheblichen Mengen von Hydroperoxiden belastet sind, müssen diese Stoffe vor der Verwendung weitgehend von Hydroperoxiden befreit werden. Das kann dadurch geschehen, daß man Hydroperoxide durch stark reduzierende Substanzen zerstört. Ein sehr geeignetes Reduktionsmittel und pharmazeutisch erlaubter Hilfsstoff ist z.B. Natriumbisulfit bzw. Natriumhydrogensulfit. In wässriger oder überwiegend wässriger Lösung können mit dieser Substanz Peroxide in schneller Reaktion problemlos zerstört werden. Leider sind jedoch die meisten für transdermale Systeme eingesetzten Polymere und Hilfsstoffe entweder nicht in Wasser löslich oder aber Wasser ist nicht kompatibel mit den anderen eingesetzten Hilfsstoffen. Überraschenderweise wurde nun ferner gefunden, daß die Zerstörung von Hydroperoxiden auch möglich ist, wenn der feste Stoff in einem mit Wasser mischbaren Lösemittel, bevorzugt Ethanol oder Methanol, gelöst wird und unter Rühren langsam mit einer wässrigen Lösung eines anorganischen Sulfites, z.B. Natriumhydrogensulfit, versetzt wird. Obwohl es bei der Zugabe zu der Lösung des Hilfsstoffs sehr schnell zu Ausfällungen kommt, haben die Sulfite noch genug Zeit, um die Hydroperoxide reduktiv zu zerstören.

Ist die Lösung des Hydrogensulfits genügend konzentriert, kann der kleine Wassereintrag meistens ohne Probleme toleriert werden. Dies gilt besonders, wenn während der Beschichtung und Trocknung das Wasser zusammen mit anderen Lösemitteln entfernt wird.

Flüssige Hilfsstoffe können auch ohne zusätzliches Lösemittel mit einer wässrigen Lösung von Natriumhydrogensulfit umgesetzt werden.

Nach dieser Behandlung sind die Materialien praktisch frei von Peroxiden und können selbst bei vorheriger erheblichen Belastung bedenkenlos eingesetzt werden. Eine zusätzliche Verbesserung der Stabilität kann dann noch erreicht werden durch den Zusatz von Antioxidantien, die die Bildung von neuen Peroxiden während der Lagerung der Systeme unterdrücken bzw. verlangsamen.

Bezüglich der tolerierbaren oberen Grenze des Peroxidgehalts, der mit dem Wirkstoff in Kontakt befindlichen Bestandteile, sollte eine Obergrenze der Peroxidzahl von 20, besser jedoch von 10, bevorzugt von 5, nicht überschritten werden.

Die Grenze von 10 ergibt sich aus folgender beispielhaften Rechnung an einem typischen transdermalen therapeutischen Matrixsystem mit einer Größe von 20 $cm^2$ und einem Beschichtungsgewicht von 100 g/ $m^2$ und einer Wirkstoffkonzentration von 10 %g/g.

Bei einem angenommenem Molekulargewicht des Wirkstoffs von 200 Dalton enthält das System demgemäß 20 mg oder 0,1 mMol Wirkstoff und bei einer Peroxidzahl von 10 insgesamt $0{,}2 \cdot 10^{-2}$ mMol aktiven Sauerstoff. Dies bedeutet, daß maximal 2 % des sich im System befindlichen Wirkstoffs oxidiert werden können. In Anbetracht der Tatsache, daß diese Reaktion Zeit braucht und sich durch den Verbrauch an aktivem Sauerstoff verlangsamt, ergibt sich bei einer Peroxidzahl von 10, und unter Umständen bei einer Obergrenze von 20, eine gute Chance, daß das System über 2 Jahre genügend stabil ist.

Eine bessere Stabilität erreicht man natürlich dadurch, daß die Peroxidbelastung weiter erniedrigt wird (bevorzugt auf eine POZ von 5 oder darunter); durch eine Behandlung der peroxidbelasteten Hilfsstoffe mit Sulfiten gemäß dem beschriebenen Verfahren bzw. durch die Wahl von Hilfsstoffen, die nicht zur Bildung von Peroxiden neigen.

**Beispiele:**

**Beispiel 1:**

Zu 0,5 g des Wirkstoffs werden 80 ml Chloroform und 1 g t-Butylhydroperoxid gegeben und unter Rühren 6 Stunden unter Rückfluß erhitzt. Danach wird die Reaktionsmischung bezüglich ihrer Farbe beurteilt bzw. mit einer geeigneten chromatographischen Methode auf gebildete Abbauprodukte untersucht.

**Beipiel 2:**

Stabilität von N-0923 Base in Matrices mit Peroxidzahl 38 und Peroxidzahl 2,6

Abbau gemäß Gleichung 4, oxidatives identifiziertes Abbauprodukt: 1,2-Dihydronapht-8-ol

| Matrix 2a: Peroxidzahl 38 | |
|---|---|
| Styrol/Polyisobutylen/Styrol-Blockpolymer | 16 % |
| Oleylalkohol | 10 % |
| Kohlenwasserstoffharz | 22 % |
| Glycerolester von partiell hydriertem Kolophonium | 22 % |
| Polyisobutylen | 7 % |
| Paraffin, flüssig | 3 % |
| N-0923 Base | 20 % |

| Matrix 2b: Peroxidzahl 2,6 | |
|---|---|
| Polyacrylatkleber | 60 % |
| Oleylalkohol | 10 % |
| N-0923 Base | 30 % |

Wirkstoffgehalt nach 3 Monaten bezogen auf den Ausgangsgehalt von 100 %

| | Matrix 2a | Matrix 2b |
|---|---|---|
| 25 °C | 85 % | 99,5 % |
| 40 °C | 44 % | 89,9 % |

Oxidatives Abbauprodukt 1,2-Dihydro-napht-8-ol in Flächenprozenten der HPLC-Chromatogramme

| | Matrix 2a | Matrix 2b |
|---|---|---|
| 25 °C | 8,1 % | nicht quantifizierbar |
| 40 °C | 34,1 % | 0,4 % |

Identifiziertes Abbauprodukt, gefunden in Reaktion mit t-Butylhydroperoxid gemäß Beispiel 1: 1,2-Dihydro-napht-8-ol

**Beispiel 3:**

Stabilität von Estradiol in Matrices mit Peroxidzahl 35 und Peroxidzahl 2

| Matrix 3a: Peroxidzahl 35 | |
|---|---|
| Polyacrylatkleber | 16 % |
| Glycerin | 10 % |
| Glycerolester von partiell hydriertem Kolophonium | 22 % |
| Estradiol | 20 % |

Matrix 3b: Peroxidzahl 2 - 3, enspricht Matrix 3a, aber Einsatz von mit Na-bisulfitlösung behandeltem Glycerolester von partiell hydriertem Kolophonium

Gehalt an Δ 9(11) 17-β-Estradiol in Matrices nach 6 Monaten, Flächenprozente in HPLC-Chromatogrammen

| | Matrix 3a | Matrix 3b |
|---|---|---|
| 25 °C | 0,43 % | nicht nachweisbar |
| 40 °C | 0,75 % | nicht nachweisbar |

**Beispiel 4:**

Stabilität von Bopindolol in peroxidreicher und peroxidarmer Matrix

| Matrixzusammensetzung: | |
|---|---|
| Bopindolol | 15 % |
| Polyacrylatkleber | 65 % |
| Glycerolester von partiell hydriertem Kolophonium | 20 % |

Matrix 4a: hergestellt mit Glycerolester von partiell hydriertem Kolophonium mit einer POZ von 160

Matrix 4b: hergestellt mit Glycerolester von partiell hydriertem Kolophonium, behandelt mit Nabisulfitlösung

| | Matrix 4a | Matrix 4b |
|---|---|---|
| 30 Tage bei 40 °C | braune Verfärbung | ohne Verfärbung |

Die Reaktion mit t-Butylhydroperoxid gemäß Beispiel 1 liefert sehr schnell eine gelbliche, dann ins stark braune übergehende Verfärbung.
Die Struktur der Abbauprodukte konnte nicht aufgeklärt werden.

**Beispiel 5:**

Stabilität von Nifedipine in peroxidreichem und peroxidarmem Reservoir

Abbauprodukte nach Oxidation mit t-Butylhydroperoxid

I.) Aromatisierung des Dihydropyridinrings gemäß Gleichung 5a
II) N-oxid-Bildung gemäß Gleichung 5b

| | |
|---|---|
| Nifedipine : | 10 % |
| Diethylenglykolmonoethylether | 90 % |

Reservoir 5a:
hergestellt mit Diethylenglykolmonoethylether mit einer POZ von 150,
**[0046]** POZdes Reservoirs: 90
Reservoir 5b:
hergestellt mit Diethylenglykolmonoethylether behandelt mit Natriumbisulfitlösung

| 30 Tage | Reservoir 5a Abbauprod. I | Reservoir5b Abbauprod. I |
|---|---|---|
| 25 °C | 1,6 % | nicht quantifizierbar |
| 40 °C | 4,5 % | 0,3 % |

Abbauprodukt II, gemäß Gleichung 5b, wurde wegen der geringen Konzentration in den Systemen nicht gefunden.

**Beispiel 6:**

Stabilität von Pergolide in peroxidreicher und peroxidarmer Matrix

Identifiziertes Abbauprodukt nach Oxidation mit t-Butylhydroperoxid:

Oxidation des Sulfidschwefels zum Sulfoxid

| Matrix 6a, POZ: ca 32 | |
|---|---|
| Pergolide | 10 % |
| Polyacrylatkleber | 70 % |
| Glycerolester von partiell hydriertem Kolophonium | 20 % |

| Matrix 6b, POZ: ca 2-3 | |
|---|---|
| Pergolide | 10 % |
| Polyacrylatkleber | 90 % |

| 30 Tage | Matrix 6a Sulfoxid | Matrix 6b Sulfoxid |
|---|---|---|
| 25 °C<br>40 °C | 0,8 %<br>4,2 % | nicht quantifizierbar<br>nicht quantifizierbar |

**Beispiel 7:**

Zerstörung von Peroxiden mit Natriumbisulfitlösung

Der zu behandelnde Rohstoff wird in einem mit Wasser mischbaren Lösemittel, bevorzugt in Methanol oder Ethanol, gelöst und unter Rühren mit einer etwa 10 - 30 prozentigen Lösung von Natriumbisulft ( Natriumhydrogensulfit ) versetzt. Die Menge an Natriumbisulfitlösung ist so bemessen, daß stöchiometrisch alle bzw. zu einem ausreichenden Grad alle Peroxide zerstört werden.

Das ausfallende Reaktionsprodukt des Natriumhydrogensulfits, Natriumhydrogensulfat, kann, wenn gewünscht oder notwendig, durch Zentrifugation oder Sedimentation oder Filtration abgetrennt werden.

## Patentansprüche

1. Transdermales therapeutisches System (TTS), worin die Bildung von oxidativen Abbauprodukten von mindestens einem, in diesem System enthaltenen und durch Hydroperoxide oxidierbaren Wirkstoff während der Lagerung des TTS verringert ist, **dadurch gekennzeichnet, daß** die Summe der Peroxidzahlen (POZ) der mit dem oder den Wirkstoff(en) in Kontakt stehenden Formulierungsbestandteilen in der durch die Rezeptur für das TTS gegebenen anteilsmäßigen Gewichtung höchstens 20 beträgt.

2. Transdermales therapeutisches System gemäß Anspruch 1, **dadurch gekennzeichnet, daß** die Summe der Peroxidzahlen höchstens 10, bevorzugt höchstens 5 beträgt.

3. Transdermales therapeutisches System gemäß einem oder mehreren der Ansprüche 1-2, **dadurch gekennzeichnet, daß** der oder die Wirkstoffe jeweils mindestens über eine sekundäre oder tertiäre Aminogruppe, eine Doppelbindung, eine CH-Bindung in Allylstellung, eine C-H-Bindung in Benzylstellung, eine tertiäre CH-Gruppe und/oder eine Sulfidgruppe verfügen.

4. Transdermales therapeutisches System gemäß einem oder mehreren der Ansprüche 1-3, **dadurch gekennzeichnet, daß** es ein ein- oder mehrschichtiges Matrixsystem ist und die den oder die Wirkstoffe enthaltende Matrix Kohlenwasserstoffharze, Polyvinylpyrrolidon oder Copolymere des Vinylpyrrolidons mit Acrylsäuren, Acrylsäurederivaten, Ethylen und/oder Vinylacetat enthält.

5. Transdermales therapeutisches System gemäß einem oder mehreren der Ansprüche 1-4, **dadurch gekennzeichnet, daß** es aus einer oder mehreren selbstklebenden wirkstoffhaltigen Schichten mit klebrigmachenden Harzen auf der Basis von Kolophoniumderivaten und/oder Polyterpenen besteht.

6. Transdermales therapeutisches System gemäß einem oder mehreren der Ansprüche 1-5, **dadurch gekennzeichnet, daß** zu den Formulierungsbestandteilen Permeationsenhancer und/oder Kristallisationsinhibitoren gehören.

7. Transdermales therapeutisches System gemäß einem oder mehreren der Ansprüche 1-4 **dadurch gekennzeichnet, daß** es ein Reservoirsystem ist und der oxidierbare Wirkstoff in einem Lösemittel oder Lösemittelgemisch gelöst ist, das über mindestens einen Ethersauerstoff, ein tertiäres Kohlenstoffatom und/oder eine CH-Gruppe in Allylstellung verfügt.

8. Transdermales therapeutisches System gemäß einem oder mehrere der Ansprüche 1-7, **dadurch gekennzeichnet, daß** als Permeationsenhancer Terpene oder Terpenderivate, ungesättigte Fettsäuren oder deren Derivate, Fettalkohole oder deren Derivate oder Diethylenglykol oder dessen Derivate verwendet werden.

9. Verfahren zur Herstellung eines transdermalen therapeutischen Systems gemäß Anspruch 1, **dadurch gekennzeichnet, daß** entweder

a) Formulierungsbestandteile ausgewählt werden, deren durch die Rezeptur für das TTS gegebenen Anteile Peroxidzahlen aufweisen, welche in der Summe eine Zahl von höchstens 20 ergeben, oder
b) hydroperoxidhaltige Formulierungsbestandteile in einer niederalkanolischen Lösung mit der wässerigen Lösung eines anorganischen Sulfits oder Hydrogensulfits behandelt werden, gegebenenfalls die ausgefallenen Reaktionsprodukte abgetrennt werden, und aus den Formulierungsbestandteilen gemäß a) oder den gemäß b) behandelten Formulierungsbestandteilen zusammen mit mindestens einem Wirkstoff auf üblichem Wege das transdermale therapeutische System hergestellt wird.

**10.** Verfahren gemäß Anspruch 9, **dadurch gekennzeichnet, daß** die festen oder flüssigen Formulierungsbestandteile in niederalkanolischer Lösung mit Natriumsulfit oder Natriumhydrogensulfit in wässriger Lösung behandelt werden.

**11.** Verfahren gemäß Anspruch 9 und 10, **dadurch gekennzeichnet, daß** die Formulierungsbestandteile in methanolischer oder ethanolischer Lösung behandelt werden.

## Claims

**1.** A transdermal therapeutic system (TTS) in which the formation of oxidative degradation products of at least one active substance present in this system and oxidizable by hydroperoxides is reduced during storage of the TTS, wherein the sum of the peroxide numbers (PON) of the formulation constituents in contact with the active substance or substances in the proportionate weighting dictated by the formula for the TTS is not more than 20.

**2.** The transdermal therapeutic system as claimed in claim 1, wherein the sum of the peroxide numbers is not more than 10, preferably not more than 5.

**3.** The transdermal therapeutic system as claimed in one or more of claims 1-2, wherein the active substance or substances each possess(es) at least one secondary or tertiary amino group, double bond, CH bond in allyl position, C-H bond in benzyl position, a tertiary CH and/or a sulfide group.

**4.** The transdermal therapeutic system as claimed in one or more of claims 1-3, which is a single-layer or multilayer matrix system and wherein the matrix comprising the active substance(s) comprises hydrocarbon resins, polyvinylpyrrolidone or copolymers of vinylpyrrolidone with acrylic acids, acrylic acid derivatives, ethylene and/or vinyl acetate.

**5.** The transdermal therapeutic system as claimed in one or more of claims 1-4, comprising one or more self-adhesive active substance layers with tackifying resins based on rosin derivatives and/or polyterpenes.

**6.** The transdermal therapeutic system as claimed in one or more of claims 1-5, wherein permeation enhancers and/or crystallization inhibitors are included in the formulation constituents.

**7.** The transdermal therapeutic system as claimed in one or more of claims 1-4, which is a reservoir system and wherein the oxidizable active substance is dissolved in a solvent or solvent mixture possessing at least one ether oxygen, tertiary carbon atom and/or CH group in allyl position.

**8.** The transdermal therapeutic system as claimed in one or more of claims 1-7, wherein permeation enhancers used comprise terpenes or terpene derivatives, unsaturated fatty acids or their derivatives, fatty alcohols or their derivatives or diethylene glycol or its derivatives.

**9.** A process for producing a transdermal therapeutic system as claimed in claim 1, which comprises either

a) selecting formulation constituents whose fractions dictated by the formula for the TTS have peroxide numbers which in total give a number of not more than 20, or
b) treating the hydroperoxide-containing formulation constituents in a lower-alkanolic solution with the aqueous solution of an inorganic sulfite or hydrogensulfite, separating off the precipitated reaction products if desired, and producing the transdermal therapeutic system by conventional means from the formulation constituents treated according to a) or the formulation constituents treated according to b) with at least one active substance.

**10.** The process as claimed in claim 9, wherein the solid or liquid formulation constituents are treated in lower-alkanolic solution with sodium sulfite or sodium hydrogen sulfite in aqueous solution.

**11.** The process as claimed in claim 9 or 10, wherein the formulation constituents are treated in methanolic or ethanolic solution.

**Revendications**

**1.** Système thérapeutique transdermique (TTS) dans lequel la formation de produits de décomposition d'oxydation d'au moins une substance active contenue dans ce système et oxydable par des hydroperoxydes pendant le stockage du TTS est diminuée, **caractérisé en ce que** la somme des indices de peroxyde (POZ) des constituants de formulation restant en contact avec la ou les substances actives dans la pondération en proportions données pour le TTS par la formulation est au maximum de 20.

**2.** Système thérapeutique transdermique selon la revendication 1, **caractérisé en ce que** la somme des indices de peroxyde s'élève au maximum à 10, de préférence au maximum à 5.

**3.** Système thérapeutique transdermique selon une ou plusieurs des revendications 1 et 2, **caractérisé en ce que** la ou les substances actives disposent respectivement d'au moins un groupe amino secondaire ou tertiaire, d'une double liaison, d'une liaison CH en position allyle, d'une liaison CH en position benzyle, d'un groupe CH tertiaire et/ou d'un groupe sulfure.

**4.** Système thérapeutique transdermique selon une ou plusieurs des revendications 1 à 3, **caractérisé en ce qu'**il est un système à matrice mono- ou multicouche et **en ce que** la matrice contenant la ou les substances actives contient des résines hydrocarbonées, de la polyvinylpyrrolidone ou des copolymères de polyvinylpyrrolidone avec des acides acryliques, des dérivés d'acide acrylique, l'éthylène et/ou l'acétate de vinyle.

**5.** Système thérapeutique transdermique selon une ou plusieurs des revendications 1 à 4, **caractérisé en ce qu'**il est constitué d'une ou plusieurs conches autocollantes contenant de la substance active avec des résines rendant adhésif à base de dérivés de colophane et/ou de polyterpènes.

**6.** Système thérapeutique transdermique selon une ou plusieurs des revendications 1 à 5, **caractérisé en ce que** des renforçateurs de perméation et/ou des inhibiteurs de cristallisation appartiennent aux constituants de la formulation.

**7.** Système thérapeutique transdermique selon une ou plusieurs des revendications 1 à 4, **caractérisé en ce qu'**il est un système à réservoir et que la substance active oxydable est dissoute dans un solvant ou un mélange de solvants, qui dispose d'au moins un oxygène en position éther, d'un atome de carbone tertiaire et/ou d'un groupe CH en position allyle.

**8.** Système thérapeutique transdermique selon une ou plusieurs des revendications 1 à 7, **caractérisé en ce qu'**on utilise comme renforçateur de perméation des terpènes ou des dérivés terpéniques, des acides gras insaturés ou leurs dérivés, des alcools gras ou leurs dérivés ou le diéthylèneglycol ou ses dérivés.

**9.** Procédé de préparation d'un système thérapeutique transdermique selon la revendication 1, **caractérisé en ce que** soit

a) on choisit des constituants de formulation dont les proportions données par la formulation pour le TTS présentent des indices de peroxyde dont il résulte une somme d'une valeur de 20 au maximum, ou
b) on traite les constituants de formulation contenant des hydroperoxydes dans une solution alcanolique inférieure avec la solution aqueuse d'un sulfite ou d'un hydrogénosulfite inorganique, on sépare éventuellement les produits de réactions précipités, et on prépare par la voie usuelle le système thérapeutique transdermique à partir des constituants de formulation selon a) ou des composés de formulation traités selon b) avec en même temps au moins une substance active.

**10.** Procédé selon la revendication 9, **caractérisé en ce qu'**on traite les constituants de formulation solides ou liquides dans une solution alcanolique inférieure avec du sulfite de sodium ou de l'hydrogénosulfite de sodium en solution

aqueuse.

**11.** Procédé selon la revendication 9 et 10, **caractérisé en ce qu'**on traite les constituants de formulation dans une solution méthanolique ou éthanolique.